# EUROPEAN PATENT APPLICATION

(11) **EP 4 249 606 A1**
(43) Date of publication of application: **27.09.2023**
(21) Application number: 22163334.0
(22) Date of filing: 21.03.2022
(51) Int. Cl.: C12Q 1/6883

(54) **PANEL OF MARKERS FOR PREDICTION OF EPILEPSY RECURRENCE IN PATIENTS WITH TUBEROUS SCLEROSIS AND THE USES THEREOF**

(71) Applicant: Warszawski Uniwersytet Medyczny, 02-091 Warszawa (PL); Instytut Biologii Doswiadczalnej PAN. im. M. Nenckiego, 02-093 Warszawa (PL); Miedzynarodowy Instytut Biologii Molekularnej I Komorkowej, 02-109 Warszawa (PL); Instytut Pomnik-Centrum Zdrowia Dziecka, 04-730 Warsaw (PL)
(72) Inventor: Roura Canalda, Adria-Jaume, 02-389 Warsaw (PL); Gielniewski, Bartlomiej, 04-382 Warsaw (PL); Jaworski, Jacek, 04-421 Warsaw (PL); Jozwiak, Sergiusz, 04-783 Warsaw (PL); Kaminska-Kaczmarek, Bozena, 05-806 Komorow (PL); Kotulska-Jozwiak, Katarzyna, 04-783 Warsaw (PL); Liszewska, Ewa, 18-400 Lomza (PL); Wojtas, Bartosz, 02-796 Warsaw (PL)
(74) Representative: Kondrat, Mariusz

(57) **Abstract**

The subject of invention is a panel of RNA markers for the prediction of epilepsy, in particular drug resistant epilepsy, in girls aged up to 24 months with tuberous sclerosis, characterised in that it comprises the following markers GLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2. Another subject of invention is an in vitro method for the prediction of epilepsy, in particular drug-resistant epilepsy, in girls aged up to 24 months with tuberous sclerosis, comprising the steps of: a) Measurement of the expression level of at least one marker; b) Determination of the risk of epilepsy, in particular drug-resistant epilepsy, based on the expression level of at least one marker, characterized in that said at least one marker is an RNA marker belonging to the panel of predictive markers according to invention, which comprises IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, wherein the prediction of the occurrence of drug-resistant epilepsy is based on a reduced expression level of said at least one marker compared to a reference level, wherein the prediction of drug-controlled epilepsy is based on elevated expression level of at least one marker compared to reference level; whereby in step (a) the expression level of at least one marker is measured before the first epileptic seizure occurs. Another subject of invention is an use of the marker panel according to invention, in the prediction of the occurrence of epilepsy, in particular drug-resistant epilepsy, in girls aged up to 24 months with tuberous sclerosis before the occurrence of epileptic seizures according to the method according to invention.

## Description

The invention relates to a panel of predictive markers, a method for the prediction of epilepsy, particularly drug-resistant epilepsy, in girls aged up to 24 months with tuberous sclerosis and the use of a panel of markers in this method.

Tuberous sclerosis (TS) also known as Tuberous Sclerosis Complex (TSC) or Bourneville-Pringle disease, belongs to the so-called neurocutaneous diseases. TSC is caused by a mutation in the TSC1 or TSC2 genes, which encode essential components of a protein complex that controls the activity of the cell metabolism regulator - mammalian target of rapamycin (mTOR). About one person in 6,000 is born with such mutation (the current number of patients worldwide is estimated to be about 1 million people). The clinical picture of TSC can vary greatly and it can present differently even in people with the same mutation. The most common manifestations of TSC include benign tumours of the brain (so-called cortical tubers or subependymal giant cell astrocytomas), skin or kidney. Approximately 90% of patients have epilepsy, often drug-resistant, occurring very early in life. The treatment of TSC is largely symptomatic and symptom-specific. In the treatment of epilepsy, drugs and approaches commonly used to treat epilepsy in infants are used. The most used drug is vigabatrin, which is, however, insufficient to achieve significant improvement in approximately 50% of patients. The need to include additional drugs (usually one or two more) defines epilepsy as drug-resistant and prolongs the treatment process, with the consequent risk of intellectual disability in the future.

The earliest possible inhibition of epilepsy is, in the case of TSC, crucial to prevent this risk, which is invaluable for the quality of life of both patients and their caregivers. This creates a need to develop new diagnostic methods to predict the development of drug-resistant epilepsy in patients with TSC before the first epileptic episodes occur.

Currently, there are no molecular markers for predicting whether epilepsy, which occurs in 90% of TSC patients, will respond to standard treatment or be drug-resistant.

The invention aimed to provide new predictive markers to predict the nature of epilepsy (drug-resistant vs. drug-controlled) in TSC patients, even before the onset of clinical seizures.

The subject of the invention is a panel of RNA markers for the prediction of epilepsy, in particular drug-resistant epilepsy, in girls aged up to 24 months with tuberous sclerosis, characterised in that it comprises the following markers GLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2.

Preferably, the panel according to the invention is extended with one or more RNA markers selected from: IGHV5-51, RRP7A, IGKV1-16, KTI12, HRAS.

Another subject of the invention is an in vitro method for the prediction of epilepsy, in particular drug-resistant epilepsy, in girls aged up to 24 months with tuberous sclerosis, comprising the following steps:
a) Measurement of the expression level of at least one marker;
b) Determination of the risk of epilepsy, in particular drug-resistant epilepsy, based on the expression level of at least one marker,
characterized in that the at least one said marker is an RNA marker belonging to a panel of predictive markers according to the invention, comprising IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, wherein predicting drug-resistant epilepsy is based on a decreased expression level of at least one of the aforementioned markers compared to baseline, and predicting drug-controlled epilepsy is based on an increased expression level of the at least one marker; wherein in step a), measuring the expression level of the at least one said marker is performed prior to the first epileptic seizure.

Preferably, the measurement is performed by qRT-PCR, ddPCR or targeted RNA sequencing by next-generation sequencing (NGS).

Preferably, the test material is peripheral blood.

Preferably, at least one marker from the prediction panel is analysed together with one or more of the following markers IGHV5-51, RRP7A, IGKV1-16, KTI12, HRAS.

Preferably, the expression level is measured and the risk of epilepsy, in particular drug-resistant epilepsy, is determined based on seven RNA markers: HRAS, RRP7A, TNFRSF13B, KTI12, IGKV4-1, IGHV6-1, IGLV1-40.

Another subject of the invention is the use of the marker panel according to the invention, in the prediction of the occurrence of epilepsy, in particular drug-resistant epilepsy, in girls aged up to 24 months with tuberous sclerosis before the occurrence of epileptic seizures according to the method according to the invention.

The invention provides the following advantages:
- The invention will allow attending physicians to better monitor the progression of the disease to select girls with TSC at the highest risk of severe epilepsy;
- The invention will allow earlier initiation of treatment with more than one antiepileptic drug, which will significantly shorten the time needed to block the disease process and thus reduce the risk of future intellectual disability of the patient.
- The panel according to the invention can be used for early diagnosis of the risk of drug-resistant epilepsy in other pediatric epilepsies.

A detailed description of the invention:
Descriptions of embodiments of the invention in the present application are provided by way of example and are not intended to limit the scope of the invention. The described embodiments comprise different features, not all of which are required in all embodiments of the invention. Some embodiments utilize only some of the features or possible combinations of the features. Variations of embodiments of the invention that are described, and embodiments of the invention comprising different combinations of features noted in the described embodiments, will come to the mind of those who are experts in the field. The scope of the invention is limited only by the claims.

Moreover, within the meaning of this description, the phrase "in an embodiment" and "in certain embodiment" is to be understood as in one or more embodiments. Further, features present in the various embodiments may be combined with each other.

In one aspect, the invention relates to a panel of RNA markers for the prediction of epilepsy, in particular drug-resistant epilepsy, in girls aged up to 24 months with tuberous sclerosis, characterised in that it comprises the following markers GLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2.

Whereby, within the meaning of the invention, a marker for prediction, also called a predictive marker, is defined as a change(s) in RNA level occurring before the onset of drug-resistant epilepsy at the clinical level. Thus, analysis using the predictive markers according to the invention must occur prior to the occurrence of clinical seizures (i.e., the patient sample must be collected prior to the occurrence of the first epileptic seizure).

The efficacy of the RNA markers according to the invention was confirmed experimentally. Patients with tuberous sclerosis (hereinafter referred to as TSC) who had not yet developed seizures were recruited for the study, which consisted of patients aged up to 24 months (mostly children at 1 year of age). Patients had blood drawn for analysis at the first visit (E0 point at 30 to 150 days until onset of clinical seizures). Patients were monitored for clinical presentation and treatment for up to 548 days after the onset of clinical seizures, after which they were assigned to a drug-responsive group (EOR) - drug-responsive epilepsy or a treatment-resistant group (EON) - drug-resistant epilepsy.

Unexpectedly, the inventors have shown that, before the onset of their first epileptic seizures, girls with TSC who develop drug-resistant epilepsy have reduced RNA levels of a marker panel comprising IGLV1-40 IGVK4-1, IGLC7, IGLV3-19, IGHV4-34, IGKV1-39, IGHV6-1, IGHV1-3, IGHV3-53, IGKV3-15, TNFRSF13B, IGLJ2, against a reference level, i.e., expression level in girls of approximately the same or the same age with TSC at the time of sampling whose future epilepsy was controlled with medication and/or against girls without TSC and of approximately the same or the same age as the female patient under study.

Girls with TSC who developed drug-controlled epilepsy before their first seizures had elevated expression levels of markers from the panel comprising IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, relative to the reference level, i.e., against girls without TSC and of similar or the same age.

Whereby the markers according to the invention are specific for female subjects.

Whereby it is understood that the panel markers according to the invention are mRNA markers.

In a preferable embodiment, the panel according to the invention is extended with one or more RNA markers selected from: IGHV5-51, RRP7A and IGKV1-16, KTI12, HRAS.

In some embodiments, the qualification of a patient with TSC as a future responder or non-responder to epilepsy treatment is based on the value of the level of the one or more RNA markers from the panel of predictive markers according to the invention, wherein the RNA level of the one or more markers from the panel must be close to the median in the group. Further, the levels should not exceed the third quartile or fall below the first quantile.

In some embodiments, one, two, three, four, five or more markers from the panel according to the invention may be used as a predictive marker.

In one embodiment, the following seven RNA markers may be used for prediction: HRAS, RRP7A, TNFRSF13B, KTI12, IGKV4-1, IGHV6-1, IGLV1-40.

In another advantageous embodiment, all the predictive markers of the panel according to the invention are used for prediction.

In some embodiments, measurement of the level of one or more predictive markers from the panel of markers according to the invention is performed in vitro using qRT-PCR, ddPCR or targeted RNA sequencing by NGS.

In some embodiments, measuring the level of one or more predictive markers from the panel of markers according to the invention is detected in vitro by, for example, radioactive assays, Southern blot, Northern blot, in situ hybridization, mass spectrometry, RT-PCR, PCR, combinations thereof, or other techniques known in the field.

In a preferred embodiment, the starting material for determining one or more predictive markers from the panel according to the invention is the peripheral blood of the patient, taken prior to the onset of clinical seizures.

In the second aspect, the invention relates to an in vitro method for the prediction of epilepsy, in particular drug-resistant epilepsy, in girls aged up to 24 months with tuberous sclerosis, comprising the steps of:
a) Measurement of the expression level of at least one marker;
b) Determination of the risk of epilepsy, in particular drug-resistant epilepsy, based on the expression level of at least one marker,
wherein at least one said marker is an RNA marker belonging to the panel of predictive markers according to invention, which comprises IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, wherein the prediction of the occurrence of drug-resistant epilepsy is based on a reduced expression level of at least one said marker compared to a reference level, wherein the prediction of drug-controlled epilepsy is based on the elevated expression level of at least one marker compared to reference level; whereby in step (a) the expression level of at least one marker is measured before the first epileptic seizure occurs.

The efficacy of the RNA markers according to the invention was confirmed experimentally. Patients with tuberous sclerosis (hereinafter referred to as TSC) who had not yet developed seizures were recruited for the study, which consisted of patients aged up to 24 months (mostly children at 1 year of age). Patients had blood drawn for analysis at the first visit (E0 point at 30 to 150 days until onset of clinical seizures). Patients were monitored for clinical presentation and treatment for up to 548 days after the onset of clinical seizures, after which they were assigned to a drug-responsive group (EOR) - drug-responsive epilepsy or a treatment-resistant group (EON) - drug-resistant epilepsy.

The inventors have shown that, before the onset of their first epileptic seizures, girls with TSC who develop drug-resistant epilepsy have reduced RNA levels of a marker panel according to the invention, i.e. - IGLV1-40 IGVK4-1, IGLC7, IGLV3-19, IGHV4-34, IGKV1-39, IGHV6-1, IGHV1-3, IGHV3-53, IGKV3-15, TNFRSF13B, IGLJ2. The reduced RNA levels of these markers were measured against a reference level. In some embodiments, the reference level was the expression in girls of approximately the same or the same age with TSC at the time of sampling whose future epilepsy was controlled with medication and/or girls without TSC and of approximately the same or the same age as the female patient under study. Wherein girls with TSC who developed drug-controlled epilepsy before their first seizures had elevated expression levels of markers from the panel comprising IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, relative to the reference level, i.e., against girls without TSC and of similar or the same age.

In some embodiments, markers of panel according to the invention are mRNA markers.

In one embodiment, the measurement of marker expression level and determination of the risk of epilepsy, including drug-resistant epilepsy, is performed based on two markers selected from the panel of predictive markers. In another embodiment, the expression level and risk of epilepsy, including drug-resistant epilepsy, is measured using three markers from the panel of predictive markers according to the invention.

In a preferable embodiment all the predictive markers of the panel according to the invention are used for prediction.

In a preferable embodiment, the panel according to the invention is extended with one or more RNA markers selected from: IGHV5-51, RRP7A and IGKV1-16, KTI12, HRAS. Which, as used in the invention, means that measurement and prediction are performed based on one or more RNA markers from a panel comprising IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, and based on one or more markers from a panel comprising IGHV5-51, RRP7A, IGKV1-16, KTI12, HRAS.

In some embodiments, the qualification of a patient with TSC as a future responder or non-responder to epilepsy treatment is based on the value of the level of the one or more RNA markers from the panel of predictive markers according to the invention, wherein the RNA level of the one or more markers from the panel must be close to the median in the group. Further, the levels should not exceed the third quartile or fall below the first quantile.

In some embodiments, in the method according to the invention, one, three, five, six, seven or more markers selected from IGLV1-40, IGVK4-1, IGLC7, IGLV3-19, IGHV4-34, IGHV5-51, IGKV1-39, IGHV6-1, IGHV1-3, IGHV3-53, IGKV3-15, RRP7A, TNFRSF13B, IGLJ2, KTI12, HRAS, IGKV1-16 may be used to predict the occurrence of epilepsy (i.e., both drug-controlled epilepsy and drug-resistant epilepsy).

In a preferable embodiment, expression level measurement and determination of the risk (i.e., prediction) of epilepsy, particularly drug drug-resistant epilepsy, is performed based on seven RNA markers: HRAS, RRP7A, TNFRSF13B, KTI12, IGKV4-1, IGHV6-1, IGLV1-40.

In some embodiments, the starting material for performing the in vitro method according to the invention is peripheral blood of the patient, collected prior to the onset of clinical seizures.

In some embodiments, in step a) of the method according to the invention, the measurement of the level of one or more predictive markers from the panel is performed by qRT-PCR, ddPCR or targeted RNA sequencing by NGS.

In other embodiments, in step a) of the method according to the invention, the measurement of one or more predictive markers from the panel is detected in vitro by, for example, Southern blot, Northern blot, in situ hybridization, mass spectrometry, RT-PCR, PCR, combinations thereof or other techniques known in the field.

In another aspect, the invention relates to the use of the marker panel according to the invention, in the prediction of the occurrence of epilepsy, in particular drug-resistant epilepsy, in girls aged up to 24 months with tuberous sclerosis before the occurrence of epileptic seizures according to the method according to the invention.

The invention is presented in exemplary embodiments and illustrated in the drawing in which: Fig. 1 is a volcano plot showing RNA differentially expressed in the patient groups E0R and E0N; Fig. 2 shows the comparison of IGLV1-40 expression in female patient groups E0R and E0N versus control group C0; Fig. 3 shows the comparison of IGVK4-1 expression in the E0R and E0N female patient groups versus group C0; Fig. 4 shows the comparison of IGLC7 expression in the E0R and E0N female patient groups versus the C0 group; Fig. 5 shows the comparison of IGLV3-19 expression in the E0R and E0N female patient groups versus group C0; Fig. 6 shows the comparison of IGHV4-34 expression in the female patient groups E0R and E0N versus group C0; Fig. 7 shows the comparison of IGHV5-51 expression in female patient groups E0R and E0N versus group C0; Fig. 8 shows the comparison of IGKV1-39 expression in the E0R and E0N female patient groups versus the C0 group; Fig. 9 shows the comparison of IGHV6-1 expression in the E0R and E0N female patient groups versus group C0; Fig. 10 shows the comparison of IGHV1-3 expression in the E0R and E0N female patient groups versus group C0; Fig. 11 shows the comparison of IGHV3-53 expression in the E0R and E0N female patient groups versus group C0; Fig. 12 shows the comparison of IGKV3-15 expression in the E0R and E0N female patient groups versus group C0; Fig. 13 shows the comparison of RRP7A expression in female patient groups E0R and E0N versus group C0; Fig. 14 shows the comparison of TNFRSF13B expression in the E0R and E0N female patient groups versus the C0 group; Fig. 15 shows the comparison of IGLJ2 expression in female patient groups E0R and E0N versus group E0; Fig. 16 shows the comparison of KTI12 expression in the E0R and E0N female patient groups versus the E0 group; Fig. 17 shows the comparison of HRAS expression in E0R and E0N female patient groups versus group C0; Fig. 18 shows the comparison of IGKV1-16 expression in the E0R and E0N female patient groups versus the C0 group.

The following exemplary embodiments are for illustrative purposes and are not intended to limit the present invention in any way.

### Example 1.

### Confirmation of the properties of the marker panel according to the invention

This example presents the methodology used to validate the selection of RNAs from a panel comprising GLV1-40, IGVK4-1, IGLC7, IGLV3-19, IGHV4-34, IGHV5-51, IGKV1-39, IGHV6-1, IGHV1-3, IGHV3-53, IGKV3-15, RRP7A, TNFRSF13B, IGLJ2, KTI12, HRAS, IGKV1-16, and RNAs from a group comprising: IGHV5-51, RRP7A, IGKV1-16, as predictive markers for drug-resistant or drug-controlled epilepsy.

### Materials and Methods

### Patients and clinical study

Patients with tuberous sclerosis (hereafter referred to as TSC) who had not yet developed seizures were recruited for the study, which consisted of patients up to 24 months of age (mostly children at 1 year of age) (n = 21) and subjects without TSC in a similar age range (n = 8). Patients had blood drawn for analysis at the first visit (E0 point at 30 to 150 days to onset of clinical seizures). Patients were monitored for clinical manifestations and type of treatment for a maximum of 548 days after the onset of clinical seizures, after which they were assigned to the E0R group - drug-responsive epilepsy or the E0N group - drug-resistant epilepsy. Fifteen patients (including 8 girls) were assigned to the E0R group and 6 (including 4 girls) to the E0N group. In addition, a group of C0 control subjects (8 individuals; 5 girls and 3 boys) of similar age but without a TSC diagnosis were studied.

### RNA Isolation

The starting material for RNA isolation was whole peripheral blood. Blood was not taken while fasting or at a specific time. 2.5 ml was collected directly into Qiagen Paxgene RNA Tubes (Qiagen; cat. no. 762165). RNA isolation was performed using the PAXgene Blood RNA Kit (50) (Qiagene; Cat. No.: 762164). The quality of RNA for RNAseq was checked by determining the RNA integrity number (RIN). This parameter was determined using an Agilent 2100 Bioanalyzer and dedicated Agilent RNA 6000 Nano Kit reagent kits. Libraries were prepared from those samples whose RIN was greater than 7 (on a 0-10 scale).

### Preparation of libraries for mRNA sequencing with rRNA depletion

The procedure for preparing libraries for RNA sequencing after ribosomal depletion was performed using the NEBNext Ultra II Directional RNA Library Prep Kit and NEBNext rRNA Depletion Kit (Human/Mouse/Rat) (NEB Ltd) according to the manufacturer's instructions. The method for rRNA depletion from the total RNA pool is based on RNAase H, which discards mitochondrial and cytoplasmic rRNA. 500 ng of total RNA was used as starting material in the procedure. The next step after rRNA depletion is thermal fragmentation of the material. In the next step, RNA is transcribed into cDNA, the first and second strand of cDNA is synthesized sequentially. The first strand of cDNA is obtained using primers that attach randomly to all regions of the RNA (random primers). In this way, a transient cDNA-RNA hybrid structure is obtained, which is then transformed into a double-stranded cDNA with simultaneous incorporation of dUTP into the second strand of cDNA. Strand specificity is obtained during complementary strand synthesis.

The resulting double-stranded cDNA was purified using AMPure XP magnetic beads. After removing the alcohol and drying the beads at room temperature, the beads were suspended in the reaction mixture for ligation. This step involves the ligation of adaptor sequences, which due to their loop structure must then be cut by the dedicated USER enzyme (NEB, Ipswich, MA, USA) to allow the final amplification of the library to occur. To obtain sufficient library concentrations, 10 cycles of PCR reactions were performed (the number of PCR cycles depends on the input RNA amount) according to the protocol in the table. Importantly, the second synthesized DNA strand containing dUTP is not amplified because the polymerase is specific only to the nucleotides present in the DNA. Consequently, this ensures that reads derived directly from transcripts are always strand-matched with R2 reads derived from paired-end sequencing.

**Table 1. PCR reaction parameters used for the final amplification of the library obtained using the NEBNext rRNA Depletion Kit.**

| **Step name** | **Temperature** | **Duration** |
|---|---|---|
| **Step I** | 98°C | 30 sec |
| **Step II (6-16 cycles)** | 98°C | 15 sec |
| | 65°C | 75 sec |
| **Step III** | 65°C | 5 min |
| **Step IV** | 4°C | ∞ |

After PCR, AMPure XP beads were added to the reaction mixture in a 1:1 ratio as described above. The library DNA was then recovered from the AMPure XP beads by eluting with 10mM Tris-HCl solution.

The final concentration of the resulting library and its purity expressed as A260/280 values (range of normal parameter values 1.8-2.2) were measured using a Nanodrop instrument (Thermo Scientific) as well as using a Quantus Fluorometer instrument and its dedicated QuantiFluor dsDNA System reagent kit (Promega, Madison, Wisconsin, USA). Library quality assessment was performed using an Agilent 2100 Bioanalyzer and a dedicated Agilent High Sensitivity DNA Kit reagent kit. Libraries were sequenced on a HiSeq1500 (Illumina, San Diego, USA) in Rapid Run paired-end mode (2x76 cycles).

### RNAseq secondary/tertiary analysis

Trimmomatic (version 0.36), with default options, was used to remove Illumina-specific adapters, low-quality 5' and 3' bases, and short reads from FASTQ files. The RNA sequencing reads were then mapped to a reference human genome sequence (hg38) using the STAR algorithm (version 2.6.1b), with the twopassMode Basic option enabled to increase read mapping to novel junctions' identification. Picard Tools (version 2.17.1) was then used to identify and mark duplicate reads. Using featureCounts software (version 1.5.3), the resulting RNAseq mapped reads were summed and counted by genes in paired (-p), reversely stranded mode (-s 2), and only reads with MAPQ values of 3 or higher were considered. For downstream analysis, samples with fewer than 10 million paired reads were deleted.

Next, low-expressed genes were pre-filtered (raw counts of at least 20 in all analyzed datasets), and the resulting RNA-seq dataset was normalized using the conditional quantile normalization method (version 1.32.0), followed by DESeq2 differentially expressed genes analysis (version 1.24.0). We used the DESeqDataSetFromMatrix function to adjust for gender effect in our first attempt to find differentially expressed genes; nevertheless, we found that the outcome was still heavily influenced by gender and not by the treatment response. As a result, we divided our data by gender and ran two separate differential expression analyses for males and females.

Standard differential expression analyses were performed using the DESeq function, with the "non-responders to treatment" group as a reference and compared with the "responders to treatment" group, considering the patients in the control group. The variance stabilizing transformation (vst) function in DESeq2 was used to transform the data for visualization reasons. Only statistically significant genes were selected (q-value ≤ 0.1), and multiple testing was controlled by the Bonferroni-Hochberg (BH) method. In addition to the degree of significance, potential biomarkers were selected based on two factors: (1) the distribution of normalized counts with emphasis onlow intra-group variability of expression, and (2) the fold change between the comparison groups, in which larger log2 FC was prioritized.

In this non-limiting example implementation, RNA sequencing was the method for measuring the amount of RNA of markers from a panel according to the invention. In contrast, within the meaning of the invention, other methods known to those familiar with the field may also be used, for example, qRT-PCR or ddPCR.

### Results

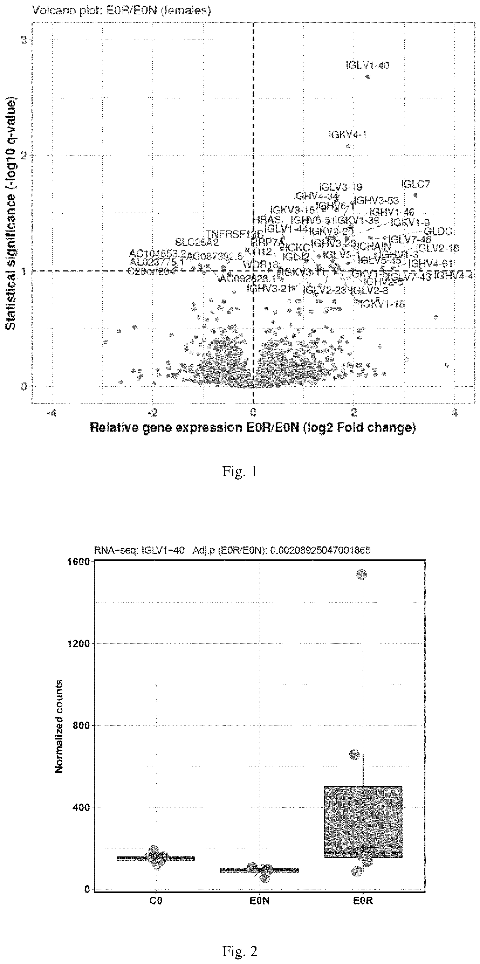

Analysis of the data performed without gender separation showed no statistically significant differences in gene expression levels at E0 between patients who subsequently developed drug-resistant epilepsy seizures (EON) compared to those whose epileptic seizures responded to treatment (E0R). Analysis by gender did not identify RNA with differential expression among boys. However, we found statistically significant differential expression of 47 RNAs in girls with TSC, as shown in Fig. 1.

This result confirms that the panel of markers, according to the invention, is specific for females.

Among all the genes studied, considering the criteria discussed above, the following RNAs were selected as potential predictive biomarkers of epilepsy, including drug-resistant epilepsy: IGLV1-40, IGVK4-1, IGLC7, IGLV3-19, IGHV4-34, IGHV5-51, IGKV1-39, IGHV6-1, IGHV1-3, IGHV3-53, IGKV3-15, RRP7A, TNFRSF13B, IGLJ2, KTI12, HRAS, IGKV1-16). Detailed results in the form of normalized counts of sequences mapped to a given RNA, statistical significance (Adj.p), and interpretation are shown in fig. 2-18 and discussed below.

### IGLV1-40 marker

As shown in fig. 2, IGLV1-40 mRNA levels in the blood of TSC female patients collected before the onset of clinical seizures are lower in those subjects who will develop drug-resistant epilepsy in the future (EON) compared to patients who will develop drug-controlled seizures (EOR) or healthy subjects (C0) defined as those without TSC and epilepsy. Low blood levels of IGLV1-40 mRNA may correlate with the occurrence of drug-resistant epilepsy and thus potentially be an indication for an early extension of standard treatment (e.g., vigabatrin for TSC) to therapeutic approaches used for drug-resistant epilepsy (hereafter referred to as aggressive treatment). In cases of drug-resistant epilepsy associated with TSC, additional antiepileptic drugs may be used as well as mTOR inhibitors, cannabidiol, ketogenic diet, and surgery.

On the other hand, the level of IGLV1-40 mRNA in the blood of TSC female patients collected before the onset of clinical seizures is higher in the E0R group than in the C0 group, which also indicates the usefulness of this marker also in the prediction of drug-controlled epilepsy.

With this non-restrictive performance example, the classification of girls with TSC aged up to 24 months into the group at risk of developing drug-resistant epilepsy or drug-controlled epilepsy based on RNA expression/quantity of a given marker assumes that the mRNA level of IGLV1-40 is close to the median in the given group. Furthermore, its level should not exceed the third quartile or fall below the first quantile.

### IGVK4-1 marker

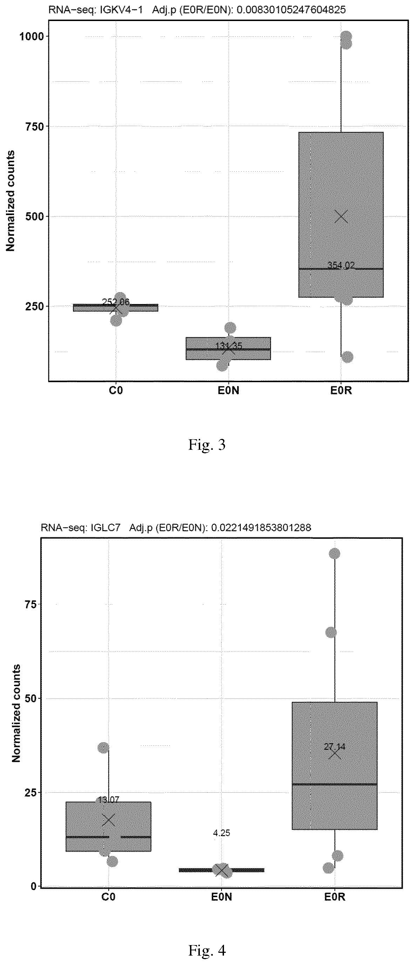

As indicated in fig. 3, IGVK4-1 mRNA levels in the blood of TSC female patients collected before the onset of clinical seizures are lower in those subjects who will develop drug-resistant epilepsy in the future (EON) compared with patients who will develop drug-controlled seizures (EOR) or healthy subjects (C0) defined as those without TSC and epilepsy. Low blood levels of IGVK4-1 mRNA may correlate with the development of drug-resistant epilepsy and thus potentially be an indication for the use of aggressive treatment.

In contrast, IGVK4-1 mRNA levels in the blood of TSC female patients collected before the onset of clinical seizures are higher in the E0R group than in the C0 group, which also indicates the usefulness of this marker in predicting drug-controlled epilepsy as well.

Classification of girls with TSC aged up to 24 months into a group at risk of developing drug-resistant epilepsy or drug-controlled epilepsy based on the expression/ RNA quantity of a given marker assumes that the IGVK4-1 mRNA level is close to the median in the given group. Furthermore, its level should not exceed the third quartile or fall below the first quantile.

### IGLC7 marker

As indicated in fig. 4, IGLC-7 mRNA levels in the blood of TSC female patients collected before the onset of clinical seizures are lower in those subjects who will develop drug-resistant epilepsy (EON) in the future compared to patients who will develop drug-controlled seizures (EOR) or healthy subjects (C0) defined as those without TSC and epilepsy. Low blood IGLC-7 mRNA levels may correlate with the development of drug-resistant epilepsy and thus potentially be an indication for the use of aggressive treatment.

In contrast, IGLC-7 mRNA levels in the blood of TSC female patients collected before the onset of clinical seizures are higher in the E0R group than in the C0 group, indicating the usefulness of this marker in predicting drug-controlled epilepsy as well.

In this implementation example, the classification of girls with TSC aged up to 24 months into the group at risk of developing drug-resistant epilepsy or drug-controlled epilepsy on the basis of the RNA expression/quantity of a given marker assumes that the IGLC-7 mRNA level is close to the median in the group. Furthermore, its level should not exceed the third quartile or fall below the first quantile.

### IGLV3-19 marker

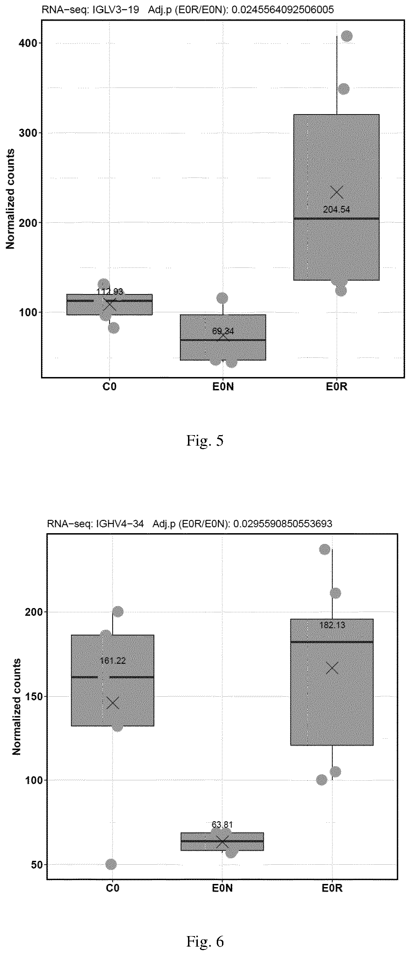

As indicated in fig. 5, the level of IGLV3-19 mRNA in the blood of TSC female patients collected before the onset of clinical seizures is lower in those subjects who will develop drug-resistant epilepsy in the future (EON) compared to patients who will develop drug-controlled seizures (EOR) or healthy subjects (C0) defined as those without TSC and epilepsy. In contrast, subjects in the E0R group have levels significantly higher than those in the C0 group. Low blood levels of IGLV3-19 mRNA may correlate with the onset of drug-resistant epilepsy and thus potentially be an indication for the inclusion of aggressive treatment while high levels of standard treatment.

In contrast, IGLV3-19 mRNA levels in the blood of TSC female patients collected before the onset of clinical seizures are higher in the E0R group than in the C0 group, also indicating the usefulness of this marker in predicting drug-controlled epilepsy as well.

In this non-limiting implementation example, the classification of girls with TSC aged up to 24 months into the group at risk of developing drug-resistant epilepsy or drug-controlled epilepsy based on RNA expression/quantity of a given marker assumes that the mRNA level of IGLV3-19 is close to the median in the group. Furthermore, its level should not exceed the third quartile or fall below the first quantile.

### IGHV4-34 marker

As indicated in fig. 6, IGHV4-34 mRNA levels in the blood of TSC female patients collected before the onset of clinical seizures are lower in those subjects who will develop drug-resistant epilepsy (EON) in the future compared to patients who will develop drug-controlled seizures (EOR) or healthy subjects (C0) defined as those without TSC and epilepsy. Low blood IGHV4-34 mRNA levels may correlate with the development of drug-resistant epilepsy and thus potentially be an indication for the use of aggressive treatment.

In contrast, IGHV4-34 mRNA levels in the blood of TSC female patients collected before the onset of clinical seizures are higher in the E0R group than in the C0 group, indicating the usefulness of this marker in predicting drug-controlled epilepsy as well.

In this implementation example, the classification of girls with TSC aged up to 24 months into a group at risk of developing drug-resistant epilepsy or drug-controlled epilepsy based on the RNA expression/quantity of a given marker assumes that the IGHV4-34 mRNA level is close to the median in the group. Furthermore, its level should not exceed the third quartile or fall below the first quantile.

### IGHV5-51 marker

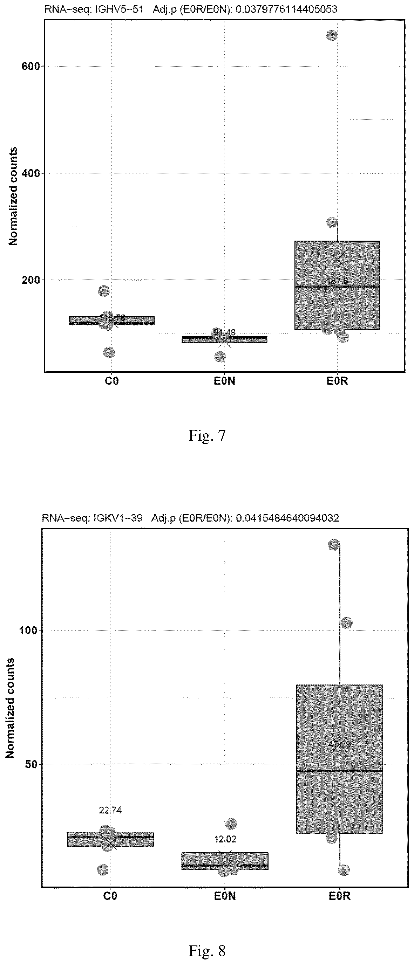

As indicated in fig. 7, IGHV5-51 mRNA levels in the blood of TSC female patients collected before the onset of clinical seizures are lower in those subjects who will develop drug-resistant epilepsy in the future (EON) compared to patients who will develop drug-controlled seizures (EOR) or healthy subjects (C0) defined as those without TSC and epilepsy. In contrast, subjects in the E0R group have levels significantly higher than those in the C0 group. Low levels of IGHV5-51 mRNA in the blood may correlate with the onset of drug-resistant epilepsy and thus potentially be an indication for inclusion of aggressive treatment, whereas high levels are an indication for inclusion of standard treatment.

In this implementation example, the classification of girls with TSC up to 24 months of age into a group predictive of the occurrence of drug-resistant epilepsy or drug-controlled epilepsy based on the RNA expression/quantity of a given marker assumes that the IGHV5-51 mRNA level is close to the median in the group. Furthermore, its level should not exceed the third quartile or fall below the first quantile.

### IGKV1-39 marker

As indicated in fig. 8, IGKV1-39 mRNA levels in the blood of TSC female patients collected before the onset of clinical seizures are lower in those subjects who will develop drug-resistant epilepsy in the future (EON) compared to patients who will develop drug-controlled seizures (EOR) or healthy subjects (C0) defined as those without TSC and epilepsy. In contrast, subjects in the E0R group have levels significantly higher than those in the C0 group. Low levels of IGKV1-39 mRNA in the blood may correlate with the onset of drug-resistant epilepsy and thus potentially be an indication for aggressive treatment, whereas high levels are an indication for standard treatment.

In this implementation example, the classification of girls with TSC aged up to 24 months into a group at risk of developing drug-resistant epilepsy or drug-controlled epilepsy based on the RNA expression/quantity of a given marker assumes that the IGKV1-39 mRNA level is close to the group median. Furthermore, its level should not exceed the third quartile or fall below the first quantile.

### IGHV6-1 marker

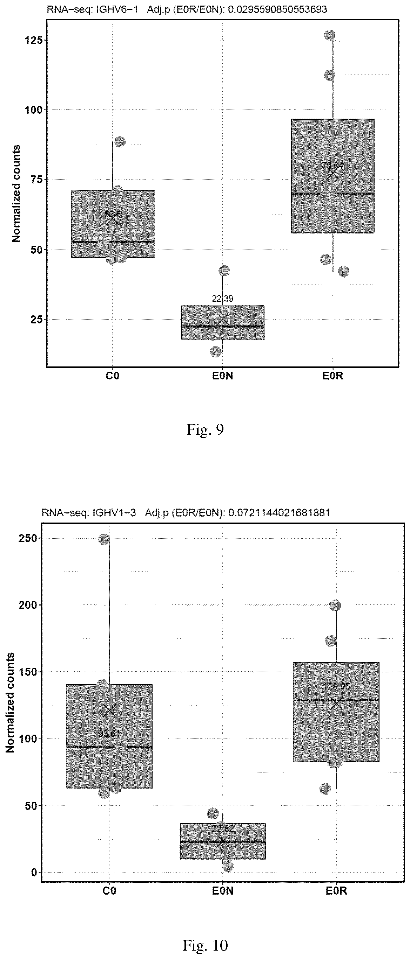

As indicated in fig. 9, IGHV6-1 mRNA levels in the blood of TSC female patients collected before the onset of clinical seizures are lower in those subjects who will develop drug-resistant epilepsy (EON) in the future compared to patients who will develop drug-controlled seizures (EOR) or healthy subjects (C0) defined as those without TSC and epilepsy. Low blood levels of IGHV6-1 mRNA may correlate with the occurrence of drug-resistant epilepsy and thus potentially be an indication for the use of aggressive treatment.

In contrast, IGHV6-1 mRNA levels in the blood of TSC patients collected before the onset of clinical seizures are higher in the E0R group than in the C0 group, indicating the usefulness of this marker in predicting drug-controlled epilepsy as well.

In this implementation example, the classification of girls with TSC aged up to 24 months into a group at risk of developing drug-resistant epilepsy or drug-controlled epilepsy based on the RNA expression/quantity of a given marker assumes that the IGHV6-1 mRNA level is close to the median in the given group. Furthermore, its level should not exceed the third quartile or fall below the first quantile.

### IGHV1-3 marker

As indicated in fig. 10, IGHV1-3 mRNA levels in the blood of TSC female patients collected before the onset of clinical seizures are lower in those subjects who will develop drug-resistant epilepsy (EON) in the future compared to patients who will develop drug-controlled seizures (EOR) or healthy subjects (C0) defined as those without TSC and epilepsy. Low blood IGHV1-3 mRNA levels may correlate with the development of drug-resistant epilepsy and thus potentially be an indication for the use of aggressive treatment.

In contrast, IGHV1-3 mRNA levels in the blood of TSC patients collected before the onset of clinical seizures are higher in the E0R group than in the C0 group, which also indicates the usefulness of this marker in predicting drug-controlled epilepsy as well.

In this non-limiting implementation example, the classification of girls with TSC aged up to 24 months into a group at risk of developing drug-resistant epilepsy or drug-controlled epilepsy based on the RNA expression/quantity of a given marker assumes that the IGHV1-3 mRNA level is close to the median in the given group. Furthermore, its level should not exceed the third quartile or fall below the first quantile.

### IGHV3-53 marker

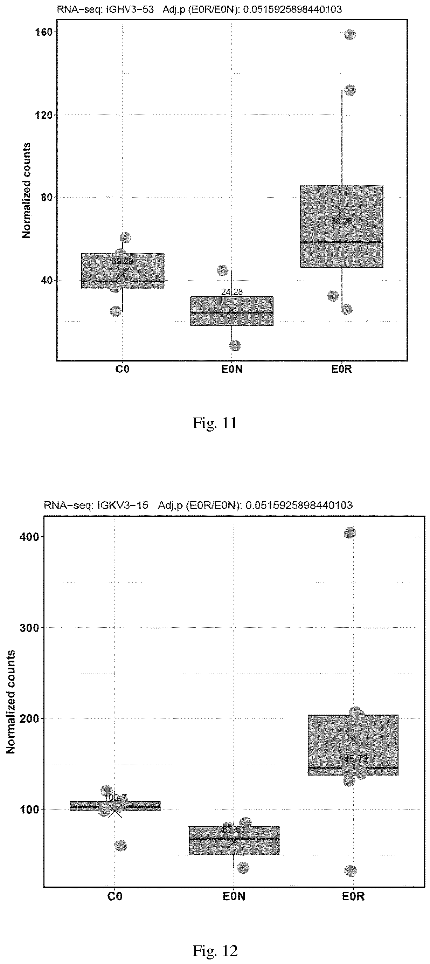

As indicated in fig. 11, IGHV3-53 mRNA levels in the blood of TSC female patients collected before the onset of clinical seizures are lower in those subjects who will develop drug-resistant epilepsy in the future (EON) compared to patients who will develop drug-controlled seizures (EOR) or healthy subjects (C0) defined as those without TSC and epilepsy. In contrast, subjects in the E0R group have levels significantly higher than those in the C0 group. Low levels of IGHV3-53 mRNA in the blood may correlate with the onset of drug-resistant epilepsy and thus potentially be an indication for starting aggressive treatment, whereas high levels are an indication for starting standard treatment.

In this non-limiting implementation example, the classification of girls with TSC aged up to 24 months into a group at risk of developing drug-resistant epilepsy or drug-controlled epilepsy based on the RNA expression/quantity of a given marker assumes that the IGHV3-53 mRNA level is close to the group median. Furthermore, its level should not exceed the third quartile or fall below the first quantile.

### IGKV3-15 marker

As indicated in fig. 12, IGKV3-15 mRNA levels in the blood of TSC female patients collected before the onset of clinical seizures are lower in those subjects who will develop drug-resistant epilepsy in the future (EON) compared to patients who will develop drug-controlled seizures (EOR) or healthy subjects (C0) defined as those without TSC and epilepsy. In contrast, subjects in the E0R group have levels significantly higher than those in the C0 group. Low blood levels of IGKV3-15 mRNA may correlate with the onset of drug-resistant epilepsy and thus potentially be an indication for aggressive treatment, whereas high levels are an indication for the use of standard treatment.

In this non-limiting implementation example, the classification of girls with TSC aged up to 24 months into a group at risk of developing drug-resistant epilepsy or drug-controlled epilepsy based on the RNA expression/quantity of a given marker assumes that the IGKV3-15 mRNA level is close to the given group median. Furthermore, its level should not exceed the third quartile or fall below the first quantile.

### RRP7A marker

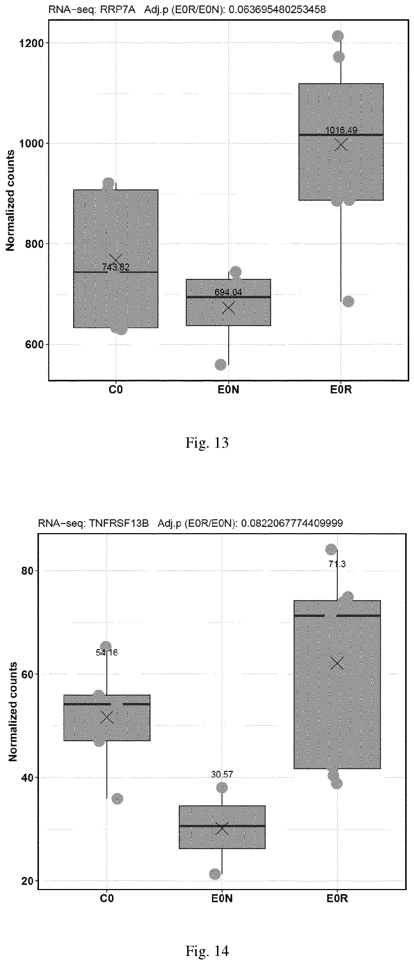

As indicated in fig. 13, the mRNA level of RRP7A in the blood of TSC female patients collected before the onset of clinical seizures is higher in those subjects who will develop drug-controlled epilepsy (EOR) in the future compared to patients who will develop drug-resistant seizures (EON) or healthy subjects (C0) defined as those without TSC and epilepsy. High blood levels of RRP7A mRNA may correlate with a lower likelihood of developing drug-resistant epilepsy and thus potentially be an indication for standard treatment.

In this implementation example, the classification of girls with TSC aged up to 24 months into a group at risk for drug-resistant epilepsy or drug-controlled epilepsy based on the RNA expression/quantity of a given marker assumes that the RRP7A mRNA level is close to the median in a given group. Furthermore, its level should not exceed the third quartile or fall below the first quantile.

### TNFRSF13B marker

As indicated in fig. 14, TNFRSF13B mRNA levels in the blood of TSC female patients collected before the onset of clinical seizures are lower in those subjects who will develop drug-resistant epilepsy (EON) in the future compared to patients who will develop drug-controlled seizures (EOR) or healthy subjects (C0) defined as those without TSC and without epilepsy. Low blood TNFRSF13B mRNA levels may correlate with the development of drug-resistant epilepsy and thus potentially be an indication for the use of aggressive treatment.

In contrast, TNFRSF13B mRNA levels in the blood of TSC patients collected before the onset of clinical seizures are higher in the E0R group than in the C0 group, also indicating the usefulness of this marker in predicting drug-controlled epilepsy as well.

In this unrestricted performance example, the classification of girls with TSC aged up to 24 months into a group at risk of developing drug-resistant epilepsy or drug-controlled epilepsy based on the RNA expression/quantity of a given marker assumes that the TNFRSF13B mRNA level is close to the median in the given group. Furthermore, its level should not exceed the third quartile or fall below the first quantile.

### IGLJ2 marker

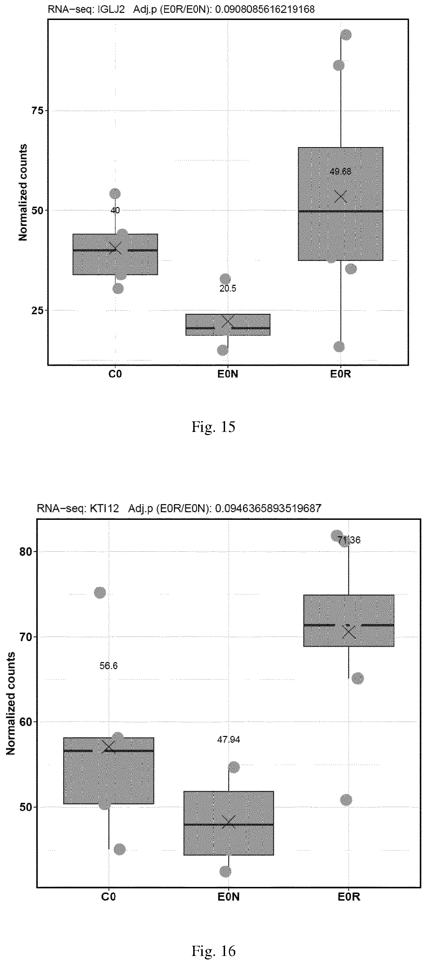

As shown in fig. 15, the mRNA level of IGLJ-2 in the blood of TSC female patients collected before the onset of clinical seizures is lower in those subjects who will develop drug-resistant epilepsy in the future (EON) compared with patients who will develop drug-controlled seizures (EOR) or healthy subjects (C0) defined as those without TSC and epilepsy. Low blood levels of IGLJ-2 mRNA may correlate with the occurrence of drug-resistant epilepsy and thus potentially be an indication for the use of aggressive treatment.

In contrast, IGLJ-2 mRNA levels in the blood of TSC patients collected before the onset of clinical seizures are higher in the E0R group than in the C0 group, which also indicates the usefulness of this marker in predicting drug-controlled epilepsy as well.

In this unrestricted implementation example, the classification of girls with TSC aged up to 24 months into the group at risk of developing drug-resistant epilepsy or drug-controlled epilepsy based on RNA expression/quantity of a given marker assumes that the mRNA level of IGLJ-2 is close to the median in the given group. Furthermore, its level should not exceed the third quartile or fall below the first quantile.

### KTI12 marker

As shown in fig. 16, the level of KTI12 mRNA in the blood of TSC female patients collected before the onset of clinical seizures is lower in those subjects who will develop drug-resistant epilepsy (EON) in the future compared to patients who will develop drug-controlled seizures (EOR) or healthy subjects (C0) defined as those without TSC and epilepsy. Low blood levels of KTI12 mRNA may correlate with the development of drug-resistant epilepsy and thus potentially be an indication for the use of aggressive treatment. In contrast, for patients with high levels (E0R), relative to the control group (C0), as well as those with refractory seizures (E0N), standard treatment would be an indication.

In this unrestricted implementation example, the classification of girls with TSC aged up to 24 months into the group at risk for drug-resistant epilepsy or drug-controlled epilepsy based on the RNA expression/quantity of a given marker assumes that the mRNA level of KTI12 is close to the median in the given group. Furthermore, its level should not exceed the third quartile or fall below the first quantile.

### HRAS marker

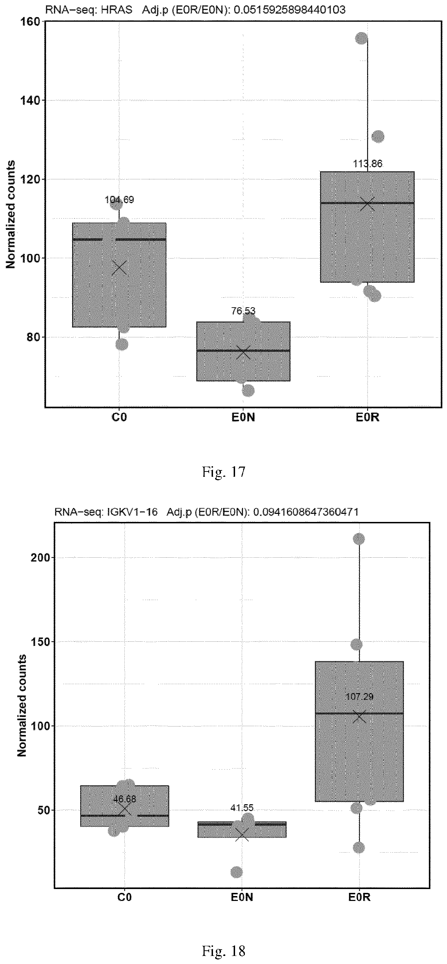

As indicated in fig. 17, HRAS mRNA levels in the blood of TSC female patients collected before the onset of clinical seizures are lower in those subjects who will develop drug-resistant epilepsy (EON) in the future compared to patients who will develop drug-controlled seizures (EOR) or healthy subjects (C0) defined as those without TSC and epilepsy. Low blood HRAS mRNA levels may correlate with the development of drug-resistant epilepsy and thus potentially be an indication for the use of aggressive treatment.

In contrast, HRAS mRNA levels in the blood of TSC patients collected before the onset of clinical seizures are higher in the E0R group than in the C0 group, which also indicates the usefulness of this marker in predicting drug-controlled epilepsy as well.

In this non-restrictive implementation example, the classification of girls with TSC aged up to 24 months into the group at risk of developing drug-resistant epilepsy or drug-controlled epilepsy based on RNA expression/quantity of a given marker assumes that the HRAS mRNA level is close to the median in the given group. Furthermore, its level should not exceed the third quartile or fall below the first quantile.

### IGKV1-16 marker

As indicated in fig. 18, IGKV1-16 mRNA levels in the blood of TSC female patients collected before the onset of clinical seizures are higher in those subjects who will develop drug-controlled epilepsy (EOR) in the future compared to patients who will develop drug-resistant seizures (EON) or healthy subjects (C0) defined as those without TSC and epilepsy. High blood levels of IGKV1-16 mRNA may correlate with the development of a milder form of epilepsy and thus potentially be an indication for the inclusion of standard treatment.

In this non-limiting implementation example, the classification of girls with TSC aged up to 24 months into a group at risk of developing drug-resistant epilepsy or drug-controlled epilepsy based on the RNA expression/quantity of a given marker assumes that the IGKV1-16 mRNA level is close to the group median. Furthermore, its level should not exceed the third quartile or fall below the first quantile.

The present analysis demonstrated the usefulness of all markers from the panel according to the invention in predicting the onset of epilepsy (including drug-resistant epilepsy and drug-controlled epilepsy) in TSC patients aged up to 24 months even before the onset of their first clinical seizures. Thus, it should be considered that the panel according to the invention will find wide application in medicine.

The use of the RNA marker panel according to the invention will allow physicians in charge to better monitor the progression of the disease in order to select girls with TSC most at risk of severe epilepsy. This would allow earlier initiation of treatment with more than one antiepileptic drug, which will significantly reduce the time needed to suppress the disease process. Furthermore, the marker panel, according to the invention, may also be helpful for early diagnosis of the risk of drug-resistant epilepsy in other childhood epilepsies.

### Example 2.

In this example, the prediction of drug-resistant epilepsy was based on the following seven RNA markers: HRAS, RRP7A, TNFRSF13B, KTI12, IGKV4-1, IGHV6-1, IGLV1-40. The patients were girls up to 24 months of age.

Collection of material from patients, isolation of RNA from peripheral blood, and sequencing were performed as in Example 1. In this example of implementation, the measurement of the expression of the markers HRAS, RRP7A, TNFRSF13B, KTI12, IGKV4-1, IGHV6-1, IGLV1-40 from the panel according to the invention was performed by RNA sequencing by NGS, while as understood in the invention the measurement of the expression of the RNA markers can also be performed by another technique known in the field, for example by qRT-PCR or ddPCR. The risk of drug-resistant epilepsy in the patients was then determined based on the levels of all seven RNA markers (i.e., HRAS, RRP7A, TNFRSF13B, KTI12, IGKV4-1, IGHV6-1, IGLV1-40) against a reference level, which was the expression level of each of these markers in the blood of girls of similar age without TSC and epileptic seizures.

In this example of implementation, the RNA markers of the panel according to the invention showed a reduced level of expression relative to the reference level, which allowed the following conclusions: the significant reduction in the level of expression of the markers TNFRSF13B, IGKV4-1, IGHV6-1, IGLV1-40 allows the examined girls to be classified as those who will develop drug-resistant epilepsy. In addition, the absence of elevated levels of markers KTI12, HRAS, RRP7A (relative to reference level) allows us to rule out that epilepsy will be drug-controlled epilepsy. Our results confirmed the efficacy of a panel of RNA markers including HRAS, RRP7A, TNFRSF13B, KTI12, IGKV4-1, IGHV6-1, IGLV1-40 in assessing the prediction of drug-resistant epilepsy.

### Example 3.

In this example, prediction of the occurrence of drug-resistant epilepsy in a girl with TSC aged up to 24 months was performed based on the following markers GLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, KTI12, HRAS, IGHV5-51, IGKV1-16.

Material collection from patients, RNA isolation from peripheral blood, and sequencing were performed as in Example 1. The risk of drug-resistant epilepsy in patients was then determined based on the level of the markers against a reference level, which was the expression level of each marker in the blood of girls of similar age without TSC and epileptic seizures.

In this example implementation, the RNA markers from the panel according to the invention showed altered expression levels relative to the reference level, which allowed the examined girls to be classified as subjects who will develop drug-resistant epilepsy due to:
significantly reduced expression levels of markers GLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, IGHV5-51 relative to the reference level,
no elevated expression of markers KTI12, HRAS, IGKV1-16 relative to the reference level.

## Claims

1. A panel of RNA markers for the prediction of epilepsy, in particular drug resistant epilepsy, in girls aged up to 24 months with tuberous sclerosis, **characterised in that** it comprises the following markers GLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2.

2. The panel according to claim 1, **characterised in that** it is extended with one or more RNA markers selected from: IGHV5-51, RRP7A, IGKV1-16, KTI12, HRAS.

3. An in vitro method for the prediction of epilepsy, in particular drug-resistant epilepsy, in girls aged up to 24 months with tuberous sclerosis, comprising the steps of:
a) Measurement of the expression level of at least one marker;
b) Determination of the risk of epilepsy, in particular drug-resistant epilepsy, based on the expression level of at least one marker,
**characterized in that** said at least one marker is an RNA marker belonging to the panel of predictive markers according to claim 1, which comprises IGLV1-40, IGVK4-1, IGLC7, IGLV3-19, IGHV4-34, IGKV1-39, IGHV6-1, IGHV1-3, IGHV3-53, IGKV3-15, TNFRSF13B, IGLJ2, wherein the prediction of the occurrence of drug-resistant epilepsy is based on a reduced expression level of said at least one marker compared to a reference level, wherein the prediction of drug-controlled epilepsy is based on elevated expression level of at least one marker compared to reference level; whereby in step (a) the expression level of at least one marker is measured before the first epileptic seizure occurs.

4. The method according to claim 3, **characterised in that** the measurement is performed by qRT-PCR, ddPCR or targeted RNA sequencing by NGS.

5. The method according to claim 3 or 4, **characterised in that** the test material is peripheral blood.

6. The method according to any of the preceding claims 3 to 5, **characterised in that** at least one marker from the prediction panel is analysed together with one or more of the following markers IGHV5-51, RRP7A, IGKV1-16, KTI12, HRAS.

7. The method according to claim 6, **characterised in that** the expression level is measured and the risk of epilepsy, in particular drug-resistant epilepsy, is determined based on seven of the following RNA markers: HRAS, RRP7A, TNFRSF13B, KTI12, IGKV4-1, IGHV6-1, IGLV1-40.

8. Use of the marker panel according to claim 1 or 2, in the prediction of the occurrence of epilepsy, in particular drug-resistant epilepsy, in girls aged up to 24 months with tuberous sclerosis before the occurrence of epileptic seizures according to the method according to any of the preceding claims 2 to 7.
